(19) Europäisches Patentamt
European Patent Office
Office européen des brevets

(11) Veröffentlichungsnummer: **0 044 444**
**B1**

# (12) EUROPÄISCHE PATENTSCHRIFT

(45) Veröffentlichungstag der Patentschrift: **25.04.84**

(51) Int. Cl.³: **C 07 C 31/20,** B 01 J 23/72, B 01 J 35/02, C 07 C 29/14

(21) Anmeldenummer: **81105027.7**

(22) Anmeldetag: **29.06.81**

## (54) Verwendung von Hydrierkatalysatoren für die Herstellung von Propandiolen und Verfahren zur Herstellung von Propandiolen mit solchen Katalysatoren.

(30) Priorität: **23.07.80 DE 3027890**

(43) Veröffentlichungstag der Anmeldung: **27.01.82 Patentblatt 82/4**

(45) Bekanntmachung des Hinweises auf die Patenterteilung: **25.04.84 Patentblatt 84/17**

(84) Benannte Vertragsstaaten: **BE CH DE FR GB LI**

(56) Entgegenhaltungen:
**EP - A - 0 006 460**
**DE - B - 2 538 253**
**GB - A - 1 371 723**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Miesen, Ernest, Samariterstrasse 3, D-6700 Ludwigshafen (DE)**
Erfinder: **Broecker, Franz Josef, Dr., Schwanthaler Allee 20, D-6700 Ludwigshafen (DE)**
Erfinder: **Schroeder, Wolfgang, Dr., Seebacher Strasse 51, D-6702 Bad Duerkheim (DE)**
Erfinder: **Baer, Karl, Dr., Kastanienweg 1, D-6940 Weinheim (DE)**
Erfinder: **Paetsch, Juergen, Dr., Am Altenbach 30, D-6706 Wachenheim (DE)**
Erfinder: **Hupfer, Leopold, Waltershoehe 3, D-6701 Friedelsheim (DE)**

**EP 0 044 444 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verwendung von Hydrierkatalysatoren für die Herstellung von Propandiolen und Verfahren zur Herstellung von Propandiolen mit solchen Katalysatoren

Die Erfindung betrifft die Verwendung von Hydrierkatalysatoren, die eine spezifische Oberfläche von 50 bis 150 Quadratmetern pro Gramm besitzen, ganz oder teilweise Kristalle mit Spinellstruktur und Kupfer in Gestalt von Kupferoxid enthalten und dadurch erhalten worden sind, daß man Kupfer und Aluminium in einem Verhältnis von 0,25 bis 3 Atomen Kupfer zu einem Atom Aluminium aus ihren Verbindungen in Gegenwart von Carbonaten bei einem pH von 4,5 bis 9 fällt und die so erhaltene Fällung bei einer Temperatur von 300 bis 800°C calciniert, zur katalytischen Herstellung von Propandiolen durch Hydrierung von Hydroxypropionaldehyden bei höchstens 300 bar und 50 bis 200°C und Verfahren zur Herstellung von Propandiolen mit solchen Katalysatoren.

Die Hydrierung von Hydroxypivalinaldehyd und anderen Propandiolen erfolgt üblicherweise in flüssiger Phase bei Drücken von 100 bis 200 bar und Temperaturen bis 150°C unter Verwendung von Nickel-, Nickel-Kupfer- und Kobaltkatalysatoren (DE-AS 1 014 089). Die DE-OS 1 804 984 beschreibt die Hydrierung bei 175 bis 220°C und 64 bis 704 bar in Gegenwart eines Kupferchromitkatalysators. In dieser Patentschrift wird darauf hingewiesen (Seite 10, letzter Absatz), daß die Wahl des eingesetzten Katalysators von entscheidender Bedeutung ist, da die Hydrierung in Gegenwart von Formaldehyd und Wasser erfolgt. Die meisten gängigen Hydrierkatalysatoren werden von Formaldehyd desaktiviert. Außerdem wird die Aktivität und Stabilität der meisten Trägermaterialien durch Wasser nachteilig beeinflußt. Mit Nickel- und Kobaltkatalysatoren werden unter den angegebenen Bedingungen unerwünschte Nebenprodukte gebildet, wodurch die Ausbeute und Reinheit des Neopentylglykols beeinträchtigt wird. Das Verfahren benötigt besondere Operationen, um das Syntheseprodukt hydrierfähig zu machen: In der ersten Aufarbeitungsstufe werden leichter siedende Anteile, Alkohole und der Isobutyraldehydüberschuß abdestilliert; in der zweiten Aufarbeitungsstufe wird der Hydroxypivalinaldehyd aus der wäßrigen, salzhaltigen Lösung mit einem organischen, in Wasser nicht löslichen Lösungsmittel extrahiert und die dabei entstehende organische Lösung des Hydroxypivalinaldehyds mit reinem Wasser salzfrei gewaschen. In der dritten Aufarbeitungsstufe wird die Lösung getrocknet. Diese Maßnahmen sind erforderlich, um den Katalysator im Hydrierreaktor vor desaktivierenden Einflüssen, besonders vor zu großer Wasserkonzentration bei der Hydrierung, zu schützen.

Der in diesem Verfahren verwendete Katalysator hat zudem den Nachteil einer vergleichsweise geringen Aktivität. So sind allein für die Hydrierung des Hydroxypivalinaldehyds in allen Beispielen eine Temperatur von 210°C und ein Wasserstoffdruck von 353 bar erforderlich. Während des Aufheizens der Hydroxypivalinaldehyd enthaltenden Lösung auf diese Temperatur bilden sich durch Tischtschenko-Reaktion Ester, die erst bei Temperaturen von 200 bis 210°C und Wasserstoffdrücken von 353 bis 423 bar hydriert werden können.

Bei einem weiteren Verfahren (britische Patentschrift 1 048 530) wird 2,2-Dimethyl-3-hydroxypropanal gleichzeitig mit Isobutyraldehyd in Gegenwart eines Kupfer-Chromoxid-Katalysators hydriert. Auch bei diesem Verfahren treten zahlreiche Nebenprodukte in deutlichem Maße auf. Wirtschaftlich einschränkend ist der Zwang, die anfallenden Mengen Isobutanol weiterzuverarbeiten. Um befriedigende Ausbeuten zu erzielen, müssen extreme Hydrierbedingungen angewandt und aufwendige Rückführungen durchgeführt werden. Ebenso aufwendig ist gegebenenfalls die Aufarbeitung bzw. die mit erheblichem Materialverlust verbundene Entsorgung der wäßrigen Phase.

Das Verfahren benötigt Hydriertemperaturen zwischen 175 und 220°C, besonders zwischen 190 und 220°C besonders zwischen 190 und 220°C und Wasserstoffdrücke zwischen 63 und 420 bar, besonders zwischen 147 und 322 bar. Diese hohen Temperaturen und Drücke sind offenbar ebenfalls wegen der bei der Reaktion gebildeten Ester erforderlich.

In einem weiteren Verfahren (DE-AS 2 054 601) verdampft man das aus einer Synthesestufe kommende, Hydroxypivalinaldehyd und niedriger siedende Lösungsmittel enthaltende Reaktionsgemisch und leitet es zusammen mit dem Hydrierwasserstoff über einen Hydrierkatalysator, der bevorzugt aus Nickel besteht. Nachteilig sind bei der Verdampfung der erforderliche Energieaufwand und die Bildung von Nebenprodukten bei diesem Verfahrensschritt.

Es ist aus der deutschen Auslegeschrift 1 957 551 bekannt, daß man Neopentylglykol durch Umsetzung von Isobutyraldehyd und Formaldehyd in Gegenwart eines basischen Katalysators und anschließender Hydrierung des gebildeten 2,2-Dimethyl-3-hydroxypropanals in Gegenwart eines Hydrierkatalysators hergestellt. Als Hydrierkatalysatoren werden Kobalt-, Kupfer-, Mangan- und/oder Nickelkatalysatoren, z. B. entsprechende Sinterkatalysatoren, genannt. Bevorzugt wird als Zusatzkomponente Phosphorsäure verwendet. In den Beispielen werden hohe Hydriertemperaturen angewandt, wodurch erst befriedigende Raum-Zeit-Ausbeuten erzielt werden. Die anzuwendenden Drücke liegen im Bereich zwischen 150 und 300 bar. Dieses Verfahren erfordert also den Einsatz teurer Hochdruckapparate sowie einen erheblichen Energieaufwand zur Kompression.

Es wurde nun gefunden, daß man Propandiole der Formel

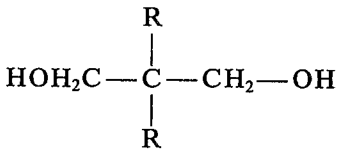

(I)

worin die einzelnen Reste R gleich oder verschieden sein können und jeweils einen aliphatischen, araliphatischen oder aromatischen Rest bedeuten, oder beide Reste R zusammen mit dem benachbarten Kohlenstoffatom Glieder eines alicyclischen Ringes bezeichnen, durch Hydrierung von Aldehyden in Gegenwart eines Kupfer enthaltenden Hydrierkatalysators vorteilhaft erhält, wenn man die Hydrierung mit Hydroxypropionaldehyden der Formel

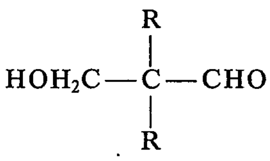

(II)

worin R die vorgenannte Bedeutung besitzt, bei einem Druck von höchstens 300 bar bei einer Hydriertemperatur von 50 bis 200°C und mit Hydrierkatalysatoren durchführt, die eine spezifische Oberfläche von 50 bis 150 Quadratmetern pro Gramm besitzen, ganz oder teilweise Kristalle mit Spinellstruktur und Kupfer in Gestalt von Kupferoxid enthalten und dadurch erhalten worden sind, daß man Kupfer und Aluminium in einem Verhältnis von 0,25 bis 3 Atomen Kupfer zu einem Atom Aluminium aus ihren Verbindungen in Gegenwart von Carbonaten bei einem pH von 4,5 bis 9 fällt und die so erhaltene Fällung bei einer Temperatur von 300 bis 800°C calciniert.

Weiterhin wurde die Verwendung der neuen Hydrierkatalysatoren zur Herstellung von Propandiolen der Formel

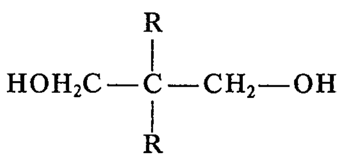

(I)

worin die einzelnen Reste R gleich oder verschieden sein können und jeweils einen aliphatischen, araliphatischen, aromatischen Rest bedeuten, oder beide Reste R zusammen mit dem benachbarten Kohlenstoffatom Glieder eines alicyclischen Ringes bezeichnen, durch Hydrierung von Hydroxypropionaldehyden der Formel

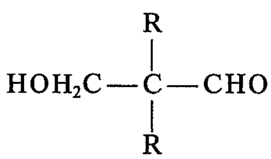

(II)

worin R die vorgenannte Bedeutung besitzt, in Gegenwart eines Kupfer enthaltenden Hydrierkatalysators, die dadurch gekennzeichnet sind, daß man die Hydrierung bei einem Druck von höchstens 300 bar bei einer Hydriertemperatur von 50 bis 200°C durchgeführt und die Hydrierkaralysatoren eine spezifische Oberfläche von 50 bis 150 Quadratmetern pro Gramm besitzen, ganz oder teilweise Kristalle mit Spinellstruktur und Kupfer in Gestalt von Kupferoxid enthalten und dadurch erhalten worden sind, daß man Kupfer und Aluminium in einem Verhältnis von 0,25 bis 3 Atomen Kupfer zu einem Atom Aluminium aus ihren Verbindungen in Gegenwart von Carbonaten bei einem pH von 4,5 bis 9 fällt und die so erhaltene Fällung bei einer Temperatur von 300 bis 800°C calciniert, gefunden.

Die Umsetzung läßt sich für den Fall der Verwendung von Hydroxypivalinaldehyd durch die folgenden Formeln wiedergeben:

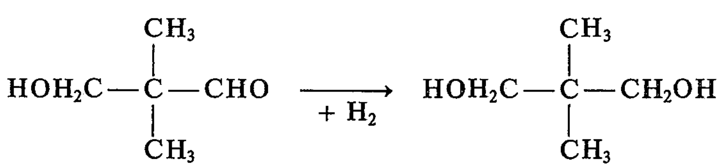


Im Hinblick auf die bekannten Verfahren liefert das Verfahren nach der Erfindung Propandiole, insbesondere Neopentylglykol, auf einfacherem und wirtschaftlicherem Wege in guter Ausbeute und

Reinheit ohne wesentliche Bildung von Nebenprodukten, z. B. Estern und Acetalen, und Zersetzungsprodukten. Zusätzliche Reinigungsoperationen oder Zusatzstoffe, die vor der Hydrierung entfernt werden müssen, sind nicht notwendig. Die hohe Hydrieraktivität des erfindungsgemäßen Katalysators ermöglicht die Hydrierung bei niedrigen Drücken und Temperaturen durchzuführen, so daß eine hydrogenolytische Spaltung z. B. des gebildeten Neopentylglykols nicht auftritt. Auch die Rückspaltung von Hydroxypivalinaldehyd in Isobutyraldehyd und Formaldehyd wird nicht beobachtet. Auch rohe Reaktionsgemische der Aldehydherstellung können direkt dem Hydrierreaktor zugeführt werden. Die Katalysatoren besitzen höhere Lebensdauer; nach 10 000 Betriebsstunden ist ihre Anfangsaktivität fast unverändert und die mechanischen Eigenschaften sind befriedigend. Teure Hochdruckapparate werden nicht benötigt. Eine Esterbildung durch Tischtschenko-Reaktion wird vermieden. Alle diese vorteilhaften Ergebnisse sind im Hinblick auf den Stand der Technik überraschend. So hätte man die hohen Raum-Zeit-Ausbeuten des erfindungsgemäßen Verfahrens im Hinblick auf die niedrigen Temperaturen und Drücke und der Gefahr damit verbundener geringerer Hydrierung bzw. Zerlegung von Nebenstoffen und damit der verstärkten Bildung heterogener Gemische nicht erwartet.

Ein wesentliches Merkmal des erfindungsgemäßen Verfahrens besteht darin, daß der Hydrierkatalysator ganz oder teilweise aus speziell strukturierten Mischkristallen hergestellt wird; diese Struktur hält bei der anschließenden thermischen Zersetzung (Calcinierung) die Feinverteilung und eine besondere Konfiguration von Kupfer und Aluminium im fertigen, d. h. calcinierten Katalysator aufrecht. Diese Konfiguration liefert die vorteilhaften erfindungsgemäßen Ergebnisse. Die durch Fällung ganz oder teilweise gebildeten Kupfer/Aluminium/OH/$CO_3$-Mischkristalle haben ein definiertes und meßbares Kristallgitter. Bevorzugt liegen die Kristalle in einer Schichtstruktur vor. Die einzelnen Gitterplätze werden von Kupfer und Aluminium sowie den Resten $CO_3$, OH und $H_2O$ eingenommen, wobei noch Zusatzstoffe, in der Regel in einer Menge von 0 bis 10, insbesondere 0 bis 5 Gewichtsprozent, bezogen auf den Gesamtkatalysator, Gitterplätze belegen können. Als Zusatzstoffe kommen zweckmäßig Chrom, Calzium, Kobalt oder Magnesium in Betracht, die in Gestalt ihrer Carbonate, Hydroxide oder Oxide als Gitterpunkte eingebaut werden oder Fehlstellen besetzen; gegebenenfalls können die Zusatzstoffe auch in amorpher oder kristalliner Form im Gemenge mit den erfindungsgemäßen Mischkristallen vorliegen.

Bei dem fertigen, calcinierten Katalysator bestehen zweckmäßig 3 bis 90 Gewichtsprozent, insbesondere 20 bis 80 Gewichtsprozent des Katalysators aus Kristallen des Spinelltyps. Bezüglich der Struktur des Spinelltyps wird auf Ullmanns Encyklopädie der technischen Chemie, Band 6 (3. Auflage, 1955) Seiten 242 bis 244, verwiesen. Die Struktur kann dem natürlichen oder synthetischen reinen Spinell wie auch den rötlichen, blauen, schwarzen, grünlichblauen, gelbgrünen, grünen, smaragdgrünen, rosaroten oder rubinfarbenen spinellen, dem spinellartigen Saphir oder dem alexandritartigen Spinell entsprechen.

Im Katalysator stehen die erfindungsgemäßen Komponenten in einem Verhältnis von 0,25 bis 3, insbesondere 0,5 bis 3 Atomen Kupfer je Atom Aluminium. Der Katalysator besitzt zweckmäßig eine spezifische Oberfläche von 50 bis 120 Quadratmetern, vorzugsweise 60 bis 120, insbesondere 60 bis 100 Quadratmetern pro Gramm, und enthält ganz oder teilweise, zweckmäßig 3 bis 50 Gewichtsprozent, insbesondere 3 bis 30 Gewichtsprozent des Gesamtkupfers in Gestalt von Kupfer-II-oxid. Die Katalysatoren haben zweckmäßig weitere spezifische Strukturmerkmale, nämlich einen mittleren Porenradius von 2000 bis 8000, vorzugsweise von 4000 bis 7000 Nanometern und ein Porenvolumen von 0,3 bis 6, vorzugsweise von 0,4 bis 1 Kubikzentimetern pro Gramm.

Bevorzugte Ausgangsstoffe II und demzufolge bevorzugte Endstoffe I sind solche, in deren Formeln die einzelnen Reste R gleich oder verschieden sind und jeweils einen Alkylrest mit 1 bis 6 Kohlenstoffatomen, einen Aralkylrest oder Alkylarylrest mit jeweils 7 bis 12 Kohlenstoffatomen, oder einen Phenylrest bedeuten, oder beide Reste R zusammen mit dem benachbarten Kohlenstoffatom Glieder eines 5- oder 6gliedrigen alicyclischen Ringes bezeichnen. Die vorgenannten Reste und Ringe können noch durch unter den Reaktionsbedingungen inerte Gruppen, z. B. Alkylgruppen oder Alkoxygruppen mit jeweils 1 bis 4 Kohlenstoffatomen, substituiert sein.

Als Ausgangsstoffe II kommen z. B. in Frage: In 2-Stellung gleich oder unterschiedlich zweifach durch Methyl-, Äthyl-, Propyl-, Isopropyl-, Butyl-, Isobutyl-, sek.-Butyl-, tert.-Butyl-, Benzyl-, Phenyl-, 2-Methylphenyl-, 3-Methylphenyl-, 4-Methylphenyl-, 3-Methoxyphenyl-, 2-Methoxyphenyl-, 4-Methoxyphenyl-gruppen substituierte 3-Hydroxypropionaldehyde; 1-Formyl-1-methylolcyclohexan, 1-Formyl-1-methylolcyclopentan.

Die Fällung wird zweckmäßig aus einer wäßrigen Lösung der Kupfersalze und Aluminiumsalze und gegebenenfalls der Zusatzstoffe mit Karbonaten und gegebenenfalls Hydroxiden durchgeführt. Als Salze kommen z. B. die Acetate, Chloride, Sulfate, Bicarbonate, Hydrogensulfate und insbesondere Nitrate von Kupfer, Aluminium und der Zusatzmetalle in Betracht. Die Carbonatreste im Katalysator können in das Fällungsbad in Gestalt entsprechender Carbonate von Kupfer, Aluminium oder Zusatzmetall, oder zweckmäßig in Gestalt von zusätzlichen, den erfindungsgemäßen pH-Wert einstellenden Alkalicarbonaten oder Alkalibicarbonaten, z. B. den Kalium- oder Natriumsalzen, zugegeben werden. Der erfindungsgemäß verwendete Katalysator wird vorteilhaft mittels zweier wäßriger Lösungen hergestellt, wobei Lösung 1 die Nitrate von Kupfer, Aluminium und gegebenenfalls

Zusatzmetall enthält. Die Lösung 2 besteht aus einer wäßrigen Natriumcarbonatlösung. Zweckmäßig verwendet man 1- bis 3molare Lösungen der Metallverbindungen und 1- bis 3molare Carbonatlösungen bzw. Bicarbonatlösungen. Im Falle der Verwendung eines Gemisches von Carbonaten und Hydroxiden, in der Regel Alkalihydroxiden, beträgt zweckmäßig der Carbonatanteil 25 bis 95 Gewichtsprozent des Gemisches. Die Fällung wird vorteilhaft bei einer Temperatur von 5 bis 90, bevorzugt 20 bis 90, zweckmäßig 40 bis 85, insbesondere 60 bis 80° C während 1 bis 2 Stunden diskontinuierlich oder kontinuierlich, unter Druck oder vorteilhaft drucklos, durchgeführt. Bevorzugt werden beide Lösungen in einen auf die Fällungstemperatur erhitzten Rührkessel geleitet. Durch Regelung der Zulaufgeschwindigkeiten wird bei dieser Parallelfällung der nachgenannte pH-Wert eingehalten. Man kann die Fällung einstufig, im allgemeinen bei einem pH von 6,9 bis 9, insbesondere 7 bis 8,5, durchführen. Zweckmäßig führt man die Fällung in 2 pH-Stufen durch, in der ersten Stufe bei 4,5 bis 6,5, bevorzugt 5 bis 6, in der zweiten Stufe bei pH 6,8 bis 9, bevorzugt 7 bis 8. Der entstandene Niederschlag wird dann zweckmäßig abfiltriert und mit Wasser salzfrei, z. B. nitratfrei, gewaschen. Eine andere Möglichkeit der Salzentfernung aus dem Niederschlag ist die Wäsche mit stark verdünnter, z. B. auf pH 10 eingestellter Natronlauge, zweckmäßig bis pH-Werte im Waschwasser zwischen 7,8 und 9,5 vor der Filtration erreicht werden. Im Falle der Verwendung von Nitraten kann man auch mit 0,1 bis 1, vorzugsweise 0,2 bis 0,3 Gewichtsprozent wäßriger $CO_2$-Lösung das Nitrat aus dem Niederschlag entfernen; hierbei geht ein Anteil von 5 bis 20 Gewichtsprozent des ausgefällten Kupfers in Lösung. Der gewaschene Niederschlag wird dann zweckmäßig bei 100 bis 120° C getrocknet, anschließend zweckmäßig geformt und dann bei 300 bis 800° C vorzugsweise 500 bis 700° C, vorteilhaft während 0,2 bis 2 Stunden, kalziniert. Bei der Formung wird der getrocknete Katalysator in die gewünschte Form gebracht, z. B. zu Tabletten oder Strängen. Man kann auch den getrockneten Katalysator z. B. während 0,1 bis 2 Stunden bei vorgenannter Temperatur calcinieren und dann verformen, z. B. zu Tabletten oder Strängen; nach der Verformung wird der so hergestellte Katalysator zweckmäßigerweise, z. B. während 0,1 bis 2 Stunden nochmals auf 200 bis 350° C erhitzt. Die Katalysatoren bedürfen in der Regel zur Entwicklung ihrer besonderen Eigenschaften nach der Filtration und der Trocknung dieser speziellen Temperaturbehandlung um ihre volle Aktivität zu entfalten, wobei eine Rekristallisation eintritt; es bildet sich Kupferoxid sowie Kristalle von Spinellstruktur, was durch Röntgenstrukturanalyse geprüft werden kann. Der Katalysator schrumpft im allgemeinen bei der Calcinierung auf 55 bis 75 Volumenprozent seines Volumens vor der Temperaturbehandlung zusammen. Die erfindungsgemäße spezifische Oberfläche bildet sich. Enthält der Katalysator $Na_2O$ oder andere Alkalimetalloxide in Mengen über 0,3 Gewichtsprozent, bezogen auf den Katalysator, so sind höhere Temperaturen, z. B. 610 bis 750° C, zweckmäßig. Bei der Herstellung von Strangpreßlingen ist es günstig, bereits calciniertes Pulver einzusetzen. Bei aluminiumärmeren Katalysatoren sind Calciniertemperaturen zwischen 300 und 600° C zweckmäßig. Der Glühverlust, bei 900° C bestimmt, liegt unter 1,5 Gewichtsprozent des Katalysators.

In einer bevorzugten Ausführungsform wird der Katalysator dann bei einer Temperatur von 150 bis 250° C mit einem Gemisch aus 0,5 bis 30 Volumenteilen Wasserstoff und 99,5 Volumenteilen bis 70 Volumenteilen Stickstoff mit 50 bis 1000 Litern Gemisch je Liter Katalysator und Stunde aktiviert. Nach der Aktivierung wird der Katalysator zweckmäßig mit Wasser oder dem Neopentylglykol enthaltenden Hydriergemisch benetzt.

Die Hydrierung des Hydroxypropionaldehyds II, insbesondere des Hydroxypivalinaldehyds, wird zweckmäßig diskontinuierlich oder bevorzugt kontinuierlich bei Temperaturen zwischen 50 und 200° C, vorzugsweise 70 bis 175° C, insbesondere 80 bis 150° C und Drücken von 1 bis 300 bar, vorzugsweise 10 bis 200 bar, insbesondere 20 bis 100 bar, durchgeführt.

Bei diskontinuierlichen Hydrierungen verwendet man den Hydrierkatalysator zweckmäßig in einer Menge von 1 bis 10, insbesondere 2 bis 5 Gewichtsprozent, bezogen auf Hydroxypropionaldehyd II, insbesondere Hydroxypivalinaldehyd, und mit Reaktionszeiten von 0,1 bis 5 Stunden. Die kontinuierliche Hydrierung erfolgt zweckmäßig nach konventionellen Techniken, z. B. in Sumpf- oder Rieselfahrweise in einem Festbettreaktor bei den genannten Temperaturen und Drücken. Man führt eine zweckmäßig wäßrige Hydroxypivalinaldehydlösung, gegebenenfalls unter Verwendung von Rückführung, unter den Vorzugsbedingungen zweckmäßig mit Hilfe von Einspritzpumpen kontinuierlich in Mengen von 0,1 bis 35 Teilen je Volumenteil Katalysator und Stunde in den Reaktor ein. Der Reaktor-Austrag wird entweder unmittelbar in eine kontinuierlich arbeitende Fraktionierkolonne eingeführt oder in Vorratsbehältern gesammelt und chargenweise fraktioniert. Man wählt im allgemeinen den Destillationsdruck so, daß der Siedepunkt des Endstoffes höher liegt als ein Schmelzpunkt (Fp 129° C), d. h. oberhalb 50 mbar.

In einer bevorzugten Ausführungsform verwendet man anstelle der Ausgangsstoffe II das Reaktionsgemisch der Herstellung von Ausgangsstoff II, z. B. anstelle von wäßriger Hydroxypivalinaldehydlösung das Reaktionsgemisch der Umsetzung von Isobutyraldehyd und Formaldehyd, zweckmäßig in einer Menge von 0,1 bis 1,5, vorteilhaft 0,9 bis 1,1 Mol Formaldehyd je Mol Isobutyraldehyd und in Gegenwart von tertiären Aminen als Katalysatoren, vorteilhaft bei einer Temperatur von 20 bis 100° C, drucklos oder unter Druck, diskontinuierlich, z. B. während 0,1 bis 4 Stunden, oder kontinuierlich.

Die nach dem Verfahren der Erfindung herstellbaren Propandiole, insbesondere 2,2-Dimethylpropandiol-(1,3) sind wertvolle Ausgangsstoffe für die Herstellung von Schmiermitteln, Kunststoffen, Lacken und Kunstharzen, z. B. entsprechenden Polyestern. Bezüglich der Verwendung wird auf die genannten Veröffentlichungen und Ullmanns Encyklopädie der technischen Chemie, Band 15, Seiten 292 ff., verwiesen.

Die in den Beispielen angegebenen Teile bedeuten Gewichtsteile. Sie verhalten sich zu den Volumenteilen wie Kilogramm zu Liter.

## Beispiel 1

### a) Herstellung des Katalysators

In einem 1. Rührkessel werden 1200 Volumenteile 27gewichtsprozentige $NaNO_3$-Lösung vorgelegt, auf 80°C erhitzt und unter Rühren bei 80°C 1200 Volumenteile pro Stunde einer wäßrigen Lösung, die 2,72 Gewichtsprozent Al und 4,8 Gewichtsprozent Cu in Gestalt ihrer Nitrate enthält, zugegeben. Gleichzeitig gibt man Anteile einer 20gewichtsprozentigen, wäßrigen Sodalösung zur Einstellung und Aufrechterhaltung eines pH von 5,5 im Rührkessel zu. Das Fällungsgemisch wird in einen zweiten Rührkessel bei 70°C gegeben und nach Eingabe von 8000 Volumenteilen Fällungsgemisch wird der pH-Wert mit 20gewichtsprozentiger Sodalösung auf 7 erhöht. Die Fällung wird während einer Stunde unter steter Einleitung von Luft oder Inertgasen gerührt, wobei der pH auf 7,9 ansteigt. Darauf wird das Gemisch filtriert und mit Wasser nitratfrei gewaschen. Die Filterpaste wird in einem Sprühturm mit Heißluft bei 120°C Ausgangstemperatur getrocknet und mit einem Gewichtsprozent Graphit als Hilfsmittel tablettiert.

Die erhaltenen Tabletten werden nun in einem beheizten Drehrohr bei einer Temperatur von 620°C während 60 Minuten calciniert. Im Drehrohr tritt eine Schrumpfung des Tablettendurchmessers von ursprünglich 4,75 auf 4,15 Millimeter ein. Der Katalysator hat ein Verhältnis von 0,75 Atomen Cu zu 1 Atom Al. Die spezifische Oberfläche beträgt 80 $m^2/g$. Die Röntgenstrukturanalyse zeigt das Gitter des CuO und eines gut ausgebildeten Spinells. Der Glühverlust beträgt 0,3 Prozent gebildeten Spinells. Der Glühverlust beträgt 0,3 Prozent bei 900°C.

### b) Hydrierung

1 Teil des nach a) dargestellten Katalysators werden in Form von Pillen in einen Hydrierreaktor eingebracht und mit 200 Volumenteilen eines Gemisches aus 5 Volumenprozent $H_2$ und 95 Volumenprozent $N_2$ mit 300 Liter Gemisch je Liter Katalysator und Stunde bei einer Temperatur von 100 bis 200°C drucklos reduziert. Nach der Aktivierung wird der Katalysator auf 110°C abgekühlt und dann aus einem Behälter stündlich 0,4 Teile einer 60gewichtsprozentigen, wäßrigen Lösung von Hydroxypivalinaldehyd (Reinheit 98%; Nebenkomponenten Isobutyraldehyd, Formaldehyd) durch den Reaktor geführt und bei 100°C und 30 bar hydriert, wobei gleichzeitig zur besseren Wärmeabfuhr und Benetzung des Katalysators 2 Teile Reaktionsaustrag im Kreise geführt werden. Die Belastung beträgt 0,24 Teile Hydroxypivalinaldehyd je Liter Katalysator und Stunde. Der Umsatz beträgt 98 Prozent. Der Hydrieraustrag wird vom Katalysator abfiltriert und über eine Glasfüllkörperkolonne fraktioniert. Man erhält aus 100 Teilen Hydrieraustrag bei 90 mbar nach Vorläufen aus Wasser- und geringen Mengen Methanol, Isobutanol und Hydroxypivalinaldehyd 54 Teile Neopentylglykol vom Siedepunkt 138 bis 140°C, entsprechend 93% der Theorie, bezogen auf den Ausgangsstoff. Der Estergehalt liegt unter 0,1 Gewichtsprozent.

## Beispiel 2

### a) Herstellung des Katalysators

Eine wäßrige Nitratlösung, die 9,8% Cu und 1,39% Al enthält, wird analog Beispiel 1 mit einer 20prozentigen Sodalösung bei pH 5,5 in einem Rührkessel mit Überlauf bei 80°C umgesetzt. Der Durchsatz beträgt 1000 Teile Nitrat-Lösung je Stunde. Die Umsetzung im zweiten Rührkessel, Aufarbeitung, Tablettierung erfolgen analog Beispiel 1a). Für die Kalzinierung wird während einer Stunde eine Temperatur von 400°C angewandt. Der Katalysator enthält Kristalle mit Spinellstruktur und Kupfer in Gestalt von Kupferoxid; die spezifische Oberfläche beträgt 80 Quadratmeter pro Gramm.

### b) Hydrierung

Ein Teil des nach a) dargestellten Katalysators wird in Form von Pillen in einen Hydrierreaktor eingebracht und mit 200 Volumenteilen eines Gemisches aus 5 Volumenprozent $H_2$ und 95

Volumenprozent $N_2$ bei einer Temperatur von 200°C mit 300 Liter Gemisch je Liter Katalysator und Stunde drucklos reduziert. Nach der Aktivierung wird der Katalysator auf 100°C abgekühlt und dann aus einem Behälter stündlich 0,3 Teile einer 60gewichtsprozentigen, wäßrigen Lösung von Hydroxypivalinaldehyd (Reinheit 98%; Nebenkomponenten Isobutyraldehyd und Formaldehyd) durch den Reaktor geführt und bei 100°C und 30 bar hydriert. Die Belastung beträgt 0,13 Teile Hydroxypivalinaldehyd je Liter Katalysator und Stunde. Der Umsatz beträgt 98 Prozent. Der Hydrieraustrag wird vom Katalysator abfiltriert und über eine Glasfüllkörperkolonne fraktioniert. Man erhält aus 100 Teilen Hydrieraustrag bei 90 mbar nach Vorläufen aus Wasser und geringen Mengen Methanol, Isobutanol und Hydroxypivalinaldehyd 54,7 Teile Neopentylglykol vom Siedepunkt 138 bis 140° C, entsprechend 93% der Theorie, bezogen auf den Ausgangsstoff. Der Estergehalt liegt unter 0,1 Gewichtsprozent.

**Patentansprüche**

1. Verwendung von Hydrierkatalysatoren zur Herstellung von Propandiolen der Formel

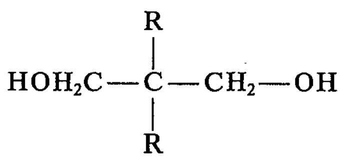

(I)

worin die einzelnen Reste R gleich oder verschieden sein können und jeweils einen aliphatischen, araliphatischen oder aromatischen Rest bedeuten, oder beide Reste R zusammen mit dem benachbarten Kohlenstoffatom Glieder eines alicyclischen Ringes bezeichnen, durch Hydrierung von Hydroxypropionaldehyden der Formel

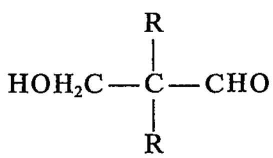

(II)

worin R die vorgenannte Bedeutung besitzt, in Gegenwart eines Kupfer enthaltenden Hydrierkatalysators, dadurch gekennzeichnet, daß man die Hydrierung bei einem Druck von höchstens 300 bar bei einer Hydriertemperatur von 50 bis 200° C durchführt und die Hydrierkatalysatoren eine spezifische Oberfläche von 50 bis 150 Quadratmetern pro Gramm besitzen, ganz oder teilweise Kristalle mit Spinellstruktur und Kupfer in Gestalt von Kupferoxid enthalten und dadurch erhalten worden sind, daß man Kupfer und Aluminium in einem Verhältnis von 0,25 bis 3 Atomen Kupfer zu einem Atom Aluminium aus ihren Verbindungen in Gegenwart von Carbonaten bei einem pH von 4,5 bis 9 fällt und die so erhaltene Fällung bei einer Temperatur von 300 bis 800° C calciniert.

2. Verfahren zur Herstellung von Propandiolen der Formel

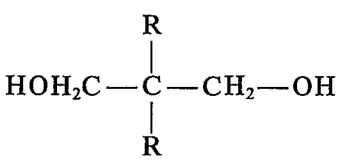

(I)

worin die einzelnen Reste R gleich oder verschieden sein können und jeweils einen aliphatischen, araliphatischen oder aromatischen Rest bedeuten, oder beide Reste R zusammen mit dem benachbarten Kohlenstoffatom Glieder eines alicyclischen Ringes bezeichnen, durch Hydrierung von Aldehyden in Gegenwart eines Kupfer enthaltenden Hydrierkatalysators, dadurch gekennzeichnet, daß man die Hydrierung mit Hydroxypropionaldehyden der Formel

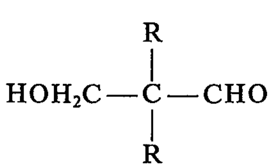

(II)

worin R die vorgenannte Bedeutung besitzt, bei einem Druck von höchstens 300 bar und bei einer Hydriertemperatur von 50 bis 200°C und mit Hydrierkatalysatoren durchführt, die eine spezifische Oberfläche von 50 bis 150 Quadratmetern pro Gramm besitzen, ganz oder teilweise Kristalle mit

Spinellstruktur und Kupfer in Gestalt von Kupferoxid enthalten und dadurch erhalten worden sind, daß man Kupfer und Aluminium in einem Verhältnis von 0,25 bis 3 Atomen Kupfer zu einem Atom Aluminium aus ihren Verbindungen in Gegenwart von Carbonaten bei einem pH von 4,5 bis 9 fällt und die so erhaltene Fällung bei einer Temperatur von 300 bis 800° C calciniert.

**Claims**

1. The use of a hydrogenation catalyst for the preparation of a propanediol of the formula

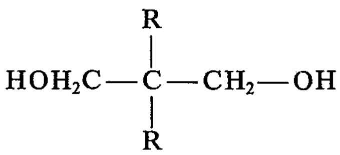 (I)

where the individual radicals R can be identical or different and each is an aliphatic, araliphatic or aromatic radical, or the two radicals R together with the adjacent carbon atom are members of an alicyclic ring, by hydrogenating a hydroxypropionaldehyde of the formula

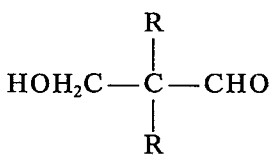 (II)

where R has the above meanings, in the presence of a copper-containing hydrogenation catalyst, wherein the hydrogenation reaction is carried out under a pressure of at most 300 bar and a hydrogenation temperature of from 50 to 200° C, and the hydrogenation catalyst has a specific surface area of from 50 to 150 square meters per gram, solely or partially contains crystals having a spinel structure and copper in the form of copper oxide, and has been obtained by precipitating copper and aluminium in a ratio of from 0.25 to 3 atoms of copper per atom of aluminium from their compounds in the presence of a carbonate at a pH of from 4.5 to 9, and calcining the resulting precipitate at from 300 to 800° C.

2. A process for the preparation of propanediols of the formula

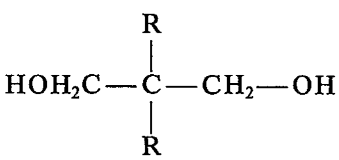 (I)

where the individual radicals R can be identical or different and each is an aliphatic, araliphatic or aromatic radical, or the two radicals R together with the adjacent carbon atom are members of an alicyclic ring, by hydrogenating an aldehyde in the presence of a copper-containing hydrogenation catalyst, wherein the hydrogenation is carried out with a hydroxypropionaldehyde of the formula

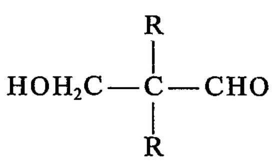 (II)

where R has the above meanings, under a pressure of at most 300 bar, at a hydrogenation temperature of from 50 to 200° C, and with a hydrogenation catalyst which has a specific surface area of from 50 to 150 square meters per gram, solely or partially contains crystals having a spinel structure and copper in the form of copper oxide, and has been obtained by precipitating copper and aluminum in a ratio of from 0.25 to 3 atoms of copper per atom of aluminum from their compounds in the presence of a carbonate at a pH of from 4.5 to 9, and calcining the resulting precipitate at from 300 to 800° C.

**Revendications**

1. Utilisation de catalyseurs d'hydrogénation pour la préparation de propane-diole de la formule

$$\begin{array}{c} \qquad\qquad R \\ \qquad\qquad | \\ HOH_2C-C-CH_2-OH \\ \qquad\qquad | \\ \qquad\qquad R \end{array} \qquad (I)$$

dans laquelle les groupes R, qui peuvent être identiques ou différents, désignent chacun un reste aliphatique, araliphatique ou aromatique, pu les deux R forment avec l'atome de carbone adjacent des chaînons d'un noyau alicyclique, par hydrogénation d'aldéhydes hydroxy-propioniques de la formule

$$\begin{array}{c} \qquad\quad R \\ \qquad\quad | \\ HOH_2C-C-CHO \\ \qquad\quad | \\ \qquad\quad R \end{array} \qquad (II)$$

dans laquelle R possède la signification définie ci-dessus, en présence d'un catalyseur d'hydrogénation au cuivre, caractérisée en ce que l'on procède à l'hydrogénation sous une pression ne dépassant pas 300 bars et à une température comprise entre 50 et 200° C en présence d'un catalyseur d'hydrogénation avec une superficie spécifique de 50 à 150 $m^2/g$, dont une partie ou la totalité renferme des cristaux de structure spinelle et qui contient du cuivre sous forme d'oxyde de cuivre, et qui a été préparé par précipitation du cuivre et de l'aluminium, dans une proportion de 0,25 à 3 atomes de cuivre pour 1 atome d'aluminium, de leurs composés en présence de carbonates et à un pH de 4,5 à 9 et calcination du précipité obtenu entre 300 et 800° C.

2. Procédé de préparation de propane-diols de la formule

$$\begin{array}{c} \qquad\qquad R \\ \qquad\qquad | \\ HOH_2C-C-CH_2-OH \\ \qquad\qquad | \\ \qquad\qquad R \end{array} \qquad (I)$$

dans laquelle les groupes R, qui peuvent être identiques ou différents, désignent chacun un reste aliphatique, araliphatique ou aromatique, ou les deux R forment avec l'atome de carbone adjacent des chaînons d'un noyau alicyclique, par hydrogénation d'aldéhydes en présence d'un catalyseur d'hydrogénation au cuivre, caractérisé en ce que l'on procède à l'hydrogénation d'aldéhydes hydroxypropioniques de la formule

$$\begin{array}{c} \qquad\quad R \\ \qquad\quad | \\ HOH_2C-C-CHO \\ \qquad\quad | \\ \qquad\quad R \end{array} \qquad (II)$$

dans laquelle R possède la signification définie cidessus, sous une pression ne dépassant pas 300 bars et à une température comprise entre 50 et 200° C en présence de catalyseurs d'hydrogénation avec une superficie spécifique de 50 à 150 $m^2/g$, dont une partie ou la totalité renferme des cristaux de structure spinelle et qui contiennent du cuivre sous forme d'oxyde de cuivre, et qui ont été préparés par précipitation du cuivre et de l'aluminium de leurs composés, dans une proportion de 0,25 à 3 atomes de cuivre pour 1 atome d'aluminium, en présence de carbonates et à un pH de 4,5 à 9, et calcination du précipité formé entre 300 et 800° C.